# EUROPEAN PATENT APPLICATION

(11) **EP 1 949 899 A1**
(43) Date of publication of application: **30.07.2008**
(21) Application number: 07100980.7
(22) Date of filing: 23.01.2007
(51) Int. Cl.: A61K 31/28, A61K 31/295, A61K 31/30, A61K 31/315, A61K 31/555, A61P 35/00, A61P 35/02, A61P 35/04

(54) **Metal salophen complexes use in prevention and treatment of cancer**

(71) Applicant: Freie Universität Berlin, 14195 Berlin (DE)
(72) Inventor: Gust, Ronald, 14513 Teltow (DE); Posselt, Diana, 13187 Berlin (DE)
(74) Representative: Ziebig, Marlene

(57) **Abstract**

The present invention relates to metal salophen complexes of general formulas I and/or II 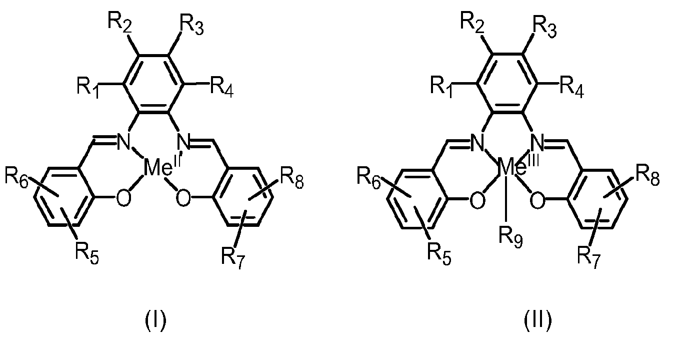 wherein
Me(II)
represents Fe, Co, Ni, Cu, Mn, Zn
Me(III)
represents Fe, Mn, AI, Cr
R₁ to R₈
independently from each other represent hydrogen, halogen, hydroxy, C₁-C₄-alkyl, C₁-C₄-alkoxy, trihalomethyl
R₉
represents halogen or acetat
or their salts or esters
for use in prevention and treatment of cancer and to pharmaceutical compositions comprising these metal salophen complexes, especially iron salophen complexes.

## Description

The present invention relates to metal salophen complexes for use in prevention or treatment of cancer and to pharmaceutical compositions comprising these metalsalophen complexes, especially iron salophen complexes.

For various reasons, cancer has become one of the major causes of death of human mainly in highly developed and industrialized countries. Several kinds of cancer have already been identified. Although not all mechanisms of the carcinogenesis and development of all kinds of cancer have been elucidated so far, cancer is a disease which is known to proceed in several steps, conventionally including the stages of initiation, promotion and progression. To prevent the proliferation of malignant tumor cells numerous anticancer drugs have been developed, among them e.g. cisplatin, tamoxifen, 5-fluorouracil, doxorubicine and adriamycin.

The technical problem underlying the present invention is to provide alternative compounds having potential to treat and prevent cancer, especially breast cancer.

The problem of the invention is solved by the provision of the embodiments as defined in the claims of the present invention. It has been found that the metal salophen complexes of general formulas I and/or II 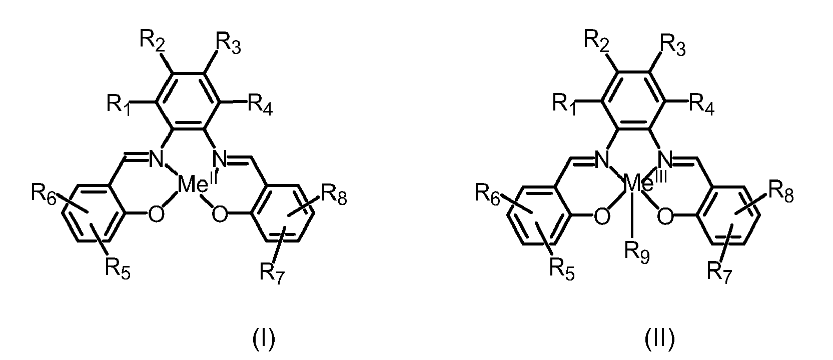 wherein
- Me(II): represents Fe, Co, Ni, Cu, Mn, Zn
- Me(III): represents Fe, Mn, Al, Cr
- R₁ to R₈: independently from each other represent hydrogen, halogen, hydroxy, C₁-C₄-alkyl, C₁-C₄-alkoxy, trihalomethyl
- R₉: represents halogen or acetat
or their salts or esters
can be used in prevention and treatment of cancer. These complexes exhibit strong cytotoxic effects in cell cultures which are superior to those of the known cytostatic drugs cisplatin, tamoxifen and 5-fluorouracil.

The term "C₁-C₄-alkyl" means methyl, ethyl, n-propyl, i-propyl, n-butyl, i-butyl, t-butyl. Preferably C₁-C₄-alkyl represents methyl or ethyl, especially preferred is methyl.

"C₁-C₄-alkoxy" denotes an alkyl group as described above bonded through an oxygen linkage (-0-). Preferably C₁-C₄-alkoxy means methoxy or ethoxy, especially preferred is methoxy.

"Trihalomethyl" means preferably trifluoro- or trichloromethyl, especially preferred is trifluoromethyl. Halogen in substituents R₁ to R₈ means F, Cl, Br, I, preferably fluorine or chlorine, especially preferred is fluorine. R₉ in formula II which also can be halogen represents chlorine, fluorine or bromine, preferably chlorine.

In an embodiment of the invention substituents R₅ to R₈ represent hydroxy and/or methoxy. It is also possible that only one of substituents R₅ and R₆ is methoxy or hydroxy and only one of substituents R₇ and R₈ is methoxy or hydroxy, whereas the remaining two substituents are hydrogen.

According to the invention the hydroxy groups in the substituents can also be protected by protecting groups well known for hydroxy like for instance benzoyl, acetyl, benzyl or p-methoxybenzyl.

In a further embodiment of the invention one of substituents R₁ to R₄ is fluorine and the remaining three substituents are hydrogen. It is also preferred that two of substituents R₁ to R₄ represent fluorine and the remaining two substituents are hydrogen. In both cases, R₅ to R₈ have either the above explained meaning or represent hydrogen or fluorine. It is also possible that only one of substituents R₅ and R₆ represents fluorine and only one of substituents R₇ and R₈ represents fluorine, whereas the remaining two substituents are hydrogen.

In a preferred embodiment of the invention Me(II) in formula I represents Fe(II). In another preferred embodiment Me(III) in formula II represents Fe(III).

In one especially preferred aspect of the invention R₁ to R₈ represent hydrogen.

Fe(II)salophen ([N,N'-bis(salicylidene)phenylene-1,2-diamine]-iron(II)) and Fe(III)salophen chloride ([N,N'-bis(salicylidene)phenylene-1,2-diamine]-iron(III)-chloride) are especially preferred complexes of the invention.

The above mentioned compounds of the invention include the pharmaceutically acceptable salts or esters, or any other compound which, upon administration to a human subject, is capable of providing (directly or indirectly) the therapeutically active metabolite.

Salts according to the invention which may be conveniently used in therapy include physiologically acceptable base salts, eg derived from an appropriate base, such as alkali metal (e.g. sodium) salts, alkaline earth metal (e.g. magnesium) salts or ammonium salts.

The complexes of formula I and II with R₁ to R₈ being hydrogen are known compounds. Their preparation is well described in the prior art. Thus, the preparation of the iron complexes is described in Sharma, Y.S. et al., Transition Metal Chemistry, 1994, 19 (3): 311-314; Earnshaw, A. et al., Journal of the Chemical Society (A), 1968: 1048-1052; Gullotti, M.et al., Journal of the Chemical Society - Dalton Transactions, 1977, 4: 339-345; Gerloch, M. et al., Journal of the Chemical Society A - Inorganic Physical Theoretical, 1968, 1: 112-116 and Nowicki, W., Transition Metal Chemistry, 1996, 21: 469-471.

The other above mentioned metal complexes of formulas I and II can be prepared in analogy to the iron complexes using the corresponding starting compounds. Such syntheses are also described in Gravert, D.J. et al., Inorganic Chemistry, 1996, 35 (17): 4837-4847 and Böttcher, A. et al., Inorganic Chemistry, 1993, 32 (19): 4131-4138.

The metal complexes of formulas I and II with substituents R₁ to R₈ other than hydrogen are synthesized in the same manner as described above using the correspondingly substituted starting compounds.

According to a further embodiment of the invention the complexes of formula I and/or II are useful for the manufacture of a pharmaceutical composition for prevention and treatment of cancer, especially of breast, colorectal, lungs, gastric, pancreatic or esophagal cancer and their metastases. In a preferred embodiment of the invention these complexes are useful for preventing and treating breast cancer.

A further proliferative disease which can be treated or prevented by the complexes of formulas I and II is a neoplasia, such as for instance leukaemia. Leukaemias include, but are not limited to, acute myelogenous leukaemia, chronic myelogenous leukaemia and juvenile myelomonocytic leukaemia.

Pharmaceutical compositions comprising in an amount sufficient to exhibit a therapeutic effect as active substance a metal salophen complex of general formulas I and/or II 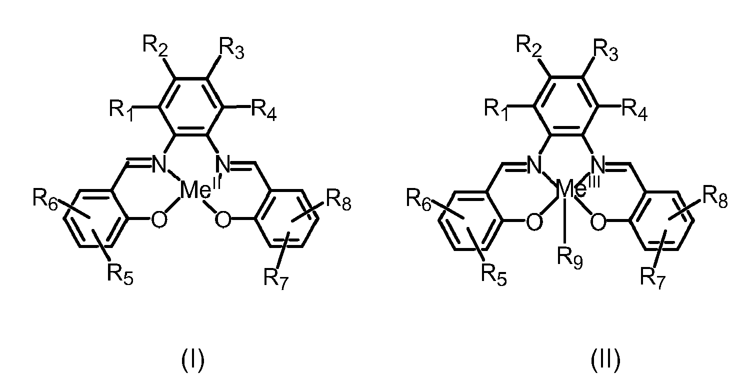 wherein
- Me(II): represents Fe, Co, Ni, Cu, Mn, Zn
- Me(III): represents Fe, Mn, Al, Cr
- R₁ to R₈: independently from each other represent hydrogen, halogen, hydroxy, C₁-C₄-alkyl, C₁-C₄-alkoxy, trihalomethyl
- R₉: represents halogen or acetat
or their salts or esters
are also an object of the present invention.

The pharmaceutical compositions may also comprise conventional auxiliary substances, preferably carriers, adjuvants and/or vehicles. For example, said carriers can be fillers, extenders, binders, humectants, disintegrants, dissolution retarders, absorption enhancers, wetting agents, adsorbents, and/or lubricants.

The pharmaceutical compositions of the invention may be prepared as a gel, powder, tablet, sustained-release tablet, premix, emulsion, infusion formulation, drops, concentrate, granulate, syrup, pellet, bolus, capsule, aerosol, spray or inhalant and/or used in this form.

For example, the pharmaceutical composition of the present invention can be administered orally in any orally tolerable dosage form, including capsules, tablets and aqueous suspensions and solutions, without being restricted thereto. In case of tablets for oral application, carriers frequently used include microcrystalline cellulose, lactose and corn starch. Typically, lubricants such as magnesium stearate can be added. For oral administration in the form of capsules, useful diluents such as lactose and dried corn starch are employed. In oral administration of aqueous suspensions the active substance is combined with emulsifiers and suspending agents. Also, particular sweeteners and/or flavors and/or coloring agents can be added, if desired.

The complexes of formulas I and/or II can also be present in micro-encapsulated form, optionally with one or more of the above-specified carriers.

In addition to the complexes of formulas I and/or II as active substance(s), suppositories may include conventional water-soluble or water-insoluble carriers such as polyethylene glycols, fats, e.g. cocoa fat and higher esters (for example, C₁₄ alcohols with C₁₆ fatty acids) or mixtures of these substances.

In addition to the complexes of formula I and/or II as active substance(s), ointments, pastes, creams and gels may include conventional carriers such as animal and vegetable fats, waxes, paraffins, starch, tragacanth, cellulose derivatives, polyethylene glycols, silicones, bentonites, silicic acid, talc and zinc oxide or mixtures of these substances.

If the pharmaceutical composition according to the invention is provided as solution or emulsion it may include conventional carriers such as solvents, solubilizers, and emulsifiers such as water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, peanut oil, corn oil, olive oil, castor oil and sesame oil, glycerol, glycerol formal, tetrahydrofurfuryl alcohol, polyethylene glycols, and fatty esters of sorbitan, or mixtures of these substances. For parenteral application, the solutions and emulsions may also be present in a sterile and blood-isotonic form.

In addition to the complexes of formulas I and/or II as active substance(s), suspensions may include conventional carriers such as liquid diluents, e.g. water, ethyl alcohol, propylene glycol, suspending agents, e.g. ethoxylated isostearyl alcohols, polyoxyethylene-sorbitol and sorbitan esters, microcrystalline cellulose, aluminum metahydroxide, bentonite, agar, and tragacanth, or mixtures of these substances.

The pharmaceutical compositions can be present in the form of a lyophilized sterile injectable formulation, e.g. as a sterile injectable aqueous solution or aqueous or oily suspension. Such a suspension can also be formulated by means of methods known in the art, using suitable dispersing or wetting agents (such as Tween 80) and suspending agents.

The production of the pharmaceutical formulations specified above proceeds in a usual manner according to well-known methods, e.g. by mixing the active substance(s) with the carrier(s).

For administration, the metal salophen complexes of formulas I and/or II may be encapsulated in semi-solid nanoparticles which prototypes are liposomes which serve as delivery systems. Liposomes are completely closed lipid bilayer membranes enclosing an aqueous volume. Liposomes can be unilamellar vesicles (i.e. having a single membrane bilayer) or multilamellar vesicles (i.e. onion-like structures characterized by several membrane bilayers each of which is separated from the next one by an aqueous layer). The production of liposomes from saturated and unsaturated lipids has been described in a large number of patents, as well as their use as delivery systems for drugs. The metal salophen complexes may be encapsulated therein in a per se known manner. Such liposomes are mostly made from phospholipids. Alternatively, the metal salophen complexes according to the invention may also be encapsulated in alginates or other gel-like structures.

According to the invention the complexes of formulas I and/or II are incorporated in a pharmaceutical formulation at a concentration of 0.1 to 99.5, preferably 0.5 to 95, and more preferably 20 to 80 wt.-%. That is, the active substance is present in the above pharmaceutical formulations, e.g. tablets, pills, granulates and others, at a concentration of preferably 0.1 to 99.5 wt.-% of the overall mixture.

According to a further embodiment the present invention provides a method for treating cancer comprising adminstering to a patient suffering from cancer a therapeutically effective and safe amount of a metal salophen complex of general formulas I and/or II 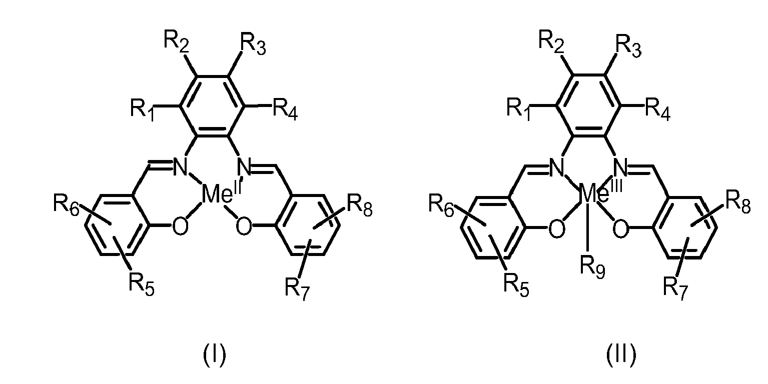 wherein
- Me(II): represents Fe, Co, Ni, Cu, Mn, Zn
- Me(III): represents Fe, Mn, Al, Cr
- R₁ to R₈: independently from each other represent hydrogen, halogen, hydroxy, C₁-C₄-alkyl, C₁-C₄-alkoxy, trihalomethyl
- R₉: represents halogen or acetat
or their salts or esters.

"Therapeutically effective amount" means an amount effective to yield the desired therapeutic response. For example, an amount effective to delay the growth of a cancer, to shrink or not metastasize. "Safe amount" refers to the quantity of a component that does not cause undue adverse side effects (such as toxicity, irritation, or allergic response) commensurate with a reasonable benefit/risk ratio when used in the manner of this invention.

Without intending to be limiting, the invention will be explained in more detail with reference to the following examples.

### Example 1: Synthesis of Fe(II)salophen and Fe(III)salophen chloride

The complexes were synthesised according to Sharma, Y.S. et al., Iron Complexes of the Schiff-Base Ligand Bis(2,5-dihydroxyacetophenone) ethylenediamine, Transition Metal Chemistry, 1994, 19 (3): 311-314.

The iron complex formation was achieved in the presence of Fe(OAc)₂ or FeCl₃. Briefly, Fe(OAc)₂ resp. FeCl₃ x 6 H₂O (0.30 mmol) was added under argon to the (salophen) ligand (0.30 mmol) in 5 ml absolute, argon-saturated ethanol. The reaction mixture immediately became dark. The solid suspension was refluxed under vigorous stirring in the presence of argon for 1 h. The mixture was then cooled to room temperature and allowed to crystallization overnight. The resulting suspension was filtered in a glove-box (filled with nitrogen), washed with small amounts of cool ethanol and dried at vacuo over P₂O₅ at room temperatur to afford the desired iron salophen complexes.

### [N,N'-Bis(salicylidene)phenylene-1,2-diamine]-iron(II) [Fe^{II}salophen]

From 0.51 mmol (160.2 mg) *N,N'*-Bis(salicylidene)phenylene-1,2-diamine and 0.52 mmol (91.2 mg) Fe(OAc)₂.
Yield: 0.48 mmol (176.1 mg), 94 %
C₂₀H₁₄FeN₂O₂ (370.18)
red-brown powder; mp: decomposion at 228 °C
**IR** (KBr): vₘₐₓ = 3433 w br; 3056 w; 1608 s; 1534 s; 1461 s; 1379 m; 1318 m; 1191 m; 921 w; 752 s; 610 m
**CHN:** calc.: C 64.89 H 3.81 N 7.57 found: C 65.13 H 3.75 N 7.82
FAB (+) (Matrix: DMSO/m-NO₂-benzyl-OH): m/z % = 393.0 [M+Na]⁺ (5); 386.0 (14); 371.0 [M+H]⁺ (57); 370 [M]^{+·} (100); 318.1 (34); 276.7 (22)

### [N,N'-Bis(salicylidene)phenylene-1,2-diamine]-iron(III)-chloride [Fe^{III}salophen]Cl

From 0.25 mmol (79.9 mg) *N,N'*-Bis(salicylidene)phenylene-1,2-diamine and 0.26 mmol (69.5 mg) FeCl₃ x 6 H₂O.
**Yield**: 0.19 mmol (78.0 mg), 74 %
C₂₀H₁₄ClFeN₂O₂x 0.5 H₂O (414.64)
dark, red-brown powder; mp: > 300 °C
**IR** (KBr): vₘₐₓ = 3432 w br; 3059 w br; 1604 s; 1578 s; 1536 s; 1461 m; 1379 m; 1312 m; 1195 m; 1150 m; 811 m; 755 m; 614 m
**CHN:** calc.: C 57.93 H 3.65 N 6.76 found: C 57.99 H 3.91 N 6.63
**FAB (+)** (Matrix: DMSO/m-NO₂-benzyl-OH): m/z % = 406.0 [M+H]⁺; 405.0 [M]^{+·} (14); 370.0 [M-Cl]⁺ (100); 276.9 (16)

### Example 2: Interaction of iron salophen complexes with DNA

### General procedure for DNA cleavage reactions with plasmid DNA on agarose gels

100 µl reaction mixture contained 1 µg of supercoiled plasmid DNA (pSK Bluescript) in a tris-HCI buffer at pH 7.0, 1 µl of iron complex solution at the desired concentration (10 mM to 10 µM in DMSO) and in some cases 1 µl of 100 µM dithiothreitol solution resp. 1 µl of 1 mM mercaptoethanol solution. After incubation at 37 °C for the indicated time, 1 µl of loading buffer (0.25 % bromophenol blue, 0,25 % xylene cyanol, 30 % glycerin in H₂O) was added to 5 µl reaction mixture (containing 50 ng plasmid DNA) and subsequently loaded onto a 0.8 % agarose gel. One µl of 1 kb-DNA-ladder (NewEnglandBiolabs, #N3232L) was loaded for estimation of size and amount of loaded DNA sample. The electrophoresis was carried out for ~ 45 min at 100 V in TBE buffer at pH 8.3. Gels were stained with etidium bromide (1 µg/ml) and photographed under 254 nm UV light.

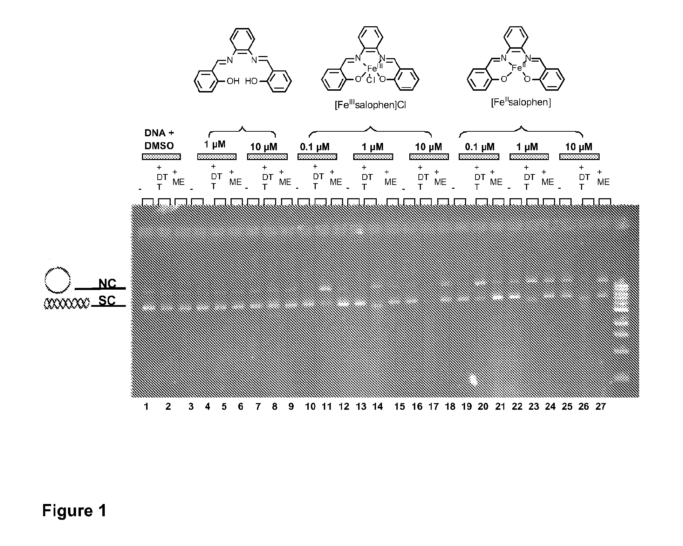

Figure 1 exhibits the gel electrophoresis of cccDNA, in pure form, in presence of [Fe^{III}salophen]CI, [Fe^{II}salophen] or its ligand after 1 h incubation at 37 °C
- without additional reducing agent
+ DTT: with 1 µM dithiothreitol
+ 2-ME: with 10 µM 2-mercaptoethanol
cccDNA: covalently closed circular DNA

From the gel in figure 1 it is apparent that the ligand (*N,N'*-bis(salicylidene)-phenylene-1,2-diamine) of the investigated iron complexes is not able to decompose DNA, neither with additional reducing agents. Fe(II)salophen and Fe(III)salophen chloride effect in low concentrations (0,1 µM) DNA single strand breakages in the presence of a reducing agent. In higher concentrations (1-10 µM) these complexes cause DNA double strand breakages. The reducing agent strengthens the DNA cleaving effect, because it causes a regeneration of Fe²⁺ which is exhausted in a Fenton reaction.

### Example 3: Accumulation of [Fe^{II} salophen] and [Fe^{III} salophen]Cl into tumor cells

The cell-uptake of iron salophen complexes was measured according to Ott, I. et al.: "Antitumor-active cobalt-alkyne complexes derived from acetylsalicylic acid: Studies on the mode of drug action", Journal of Medicinal Chemistry, 2005, 48 (2): 622-629 and Ott, I. et al.: "Investigations on the effects of cobalt-alkyne complexes on leukemia and lymphoma cells: cytotoxicity and cellular uptake", 2004, Journal of Inorganic Biochemistry 98 (3): 485-489.

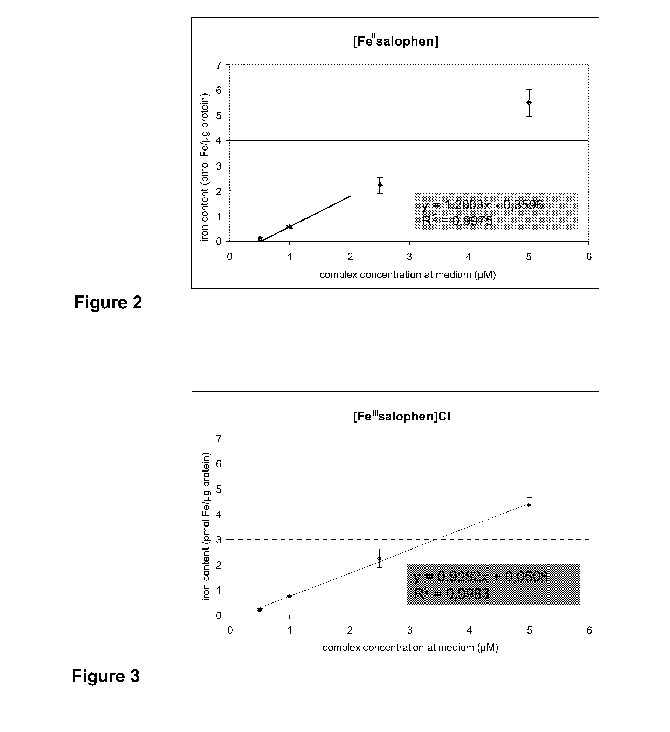

The calculation of intracellular iron content was performed according to Gust, R. et al.: "Stability and Cellular Studies of [rac-1,2-Bis(4-fluorophenyl)-ethylenediamine][cyclobutane-1,1-dicarboxylato]platinum(II), a Novel, Highly Active Carboplatin Derivative", Journal of Cancer Research and Clinical Oncology, 1998, 124 (11): 585-597 and the protein concentration was measured according to Bradford, M.M.: "Rapid and Sensitive Method for Quantitation of Microgram Quantities of Protein Utilizing Principle of Protein-Dye Binding". Analytical Biochemistry, 1976, 72 (1-2): 248-254.
The results are shown in figures 2 and 3 and in the following tables 1 and 2. Figure 2 exhibits the concentration dependent accumulation of Fe^{II}[salophen] into MCF-7 cells (Incubation time 24 h, n = 6) and Figure 3 exhibits the concentration dependent accumulation of Fe^{III}[salophen]Cl into MCF-7 cells (Incubation time 24 h, n = 6)

**Table 1: Cell-uptake of iron complexes in MCF-7 breast cancer cells after an incubation time of 24 hours and with a test concentration of 5 µM**

| | **MCF-7 (µM)** | |
|---|---|---|
| **Compound** | **intracellular iron content (µM)** | **degree of enrichment** |
| Fe(II)salophen | 588.0 ± 30.7 | 117.6 ± 6.1 |
| Fe(III)salophen chloride | 497.4 ± 28.2 | 99.5 ± 5.6 |

**Table 2: Cell-uptake of iron complexes in MDA-MB-231 breast cancer cells after an incubation time of 24 hours and with a test concentration of 5 µM**

| | **MDA-MB 231 (µM)** | |
|---|---|---|
| **Compound** | **intracellular iron content (µM)** | **degree of enrichment** |
| Fe(II)salophen | 703.2 (± 6.8) | 140.7 (± 1.4) |
| Fe(III)salophen chloride | 731.3 (±72.1) | 146.3 (±14.4) |

As it can be taken from tables 1 and 2, at a test concentration of 5 µM both compounds are enriched in MCF-7 cells hundred fold in comparison to the outer medium. In MDA-MB 231 cells the compounds are accumulated 140-fold. That means, for the iron complexes of the invention the found degree of enrichment in tumor cells is considerably higher than that of the known platin complexes which are in clinical use. As described by Ghezzi, A. et al., Journal of Inorganic Biochemistry, 2004, 98 (1): 73-78 cisplatin is enriched in MCF-7 cells fivefold in comparison with the outer medium. For oxaliplatin a sixfold enrichment has been found and for carboplatin an enrichment of merely 1,6 has been established.

### Example 4: Determination of cytotoxic effects of Fe(II)salophen and Fe(III)salophen chloride

### a) Concentration dependent test (IC₅₀)

Measurement of IC₅₀-values was performed as described by Ott, I. et al., Journal of Medicinal Chemistry, 2005, 48 (2): 622-629. But unlike described in the literature, the experiments with MCF-7-cells were stopped after 96 hours and experiments with MDA-MB-231-cells were stopped after 72 hours.

### b) Time dependent test

The time dependent tests were performed according to Reile, H. et al.: "Computerized Determination of Growth Kinetic Curves and Doubling Times from Cells in Microculture", Analytical Biochemistry, 1990, 187 (2): 262-267; Bernhardt, G. et al.: "Standardized kinetic microassay to quantifiy differential chemosensitivity on the basis of proliferative activity", Journal of Research Clin. Oncol., 1992, 118: 35-43 and Gust, R. et al.: "Development of Cobalt(3,4-diarylsalen) Complexes as Tumor Therapeutics", Journal of Medicinal Chemistry, 2004, 47 (24): 5837-5846.

| **cell line** | **seed density (cells/vial)** | **incubation time** |
|---|---|---|
| MCF-7 | 700 - 720 | ca. 72 h |
| MDA-MB-231 | 320 - 330 | ca. 52 h |
| LNCaP/FGC | 1000 - 1100 | ca. 96 h |
| HeLa | 320 - 330 | ca. 52 h |

Determining the cell mass in the crystal violet assay the interpretation of the received values was the following:

| | | | | |
|---|---|---|---|---|
| | T/C_{corr} | | > 80 % | no antiproliferative effect |
| 80 % > | T/C_{corr} | | ≥ 20 % | antiproliferative effect |
| 20 % > | T/C_{corr} | | ≥ 0 % | cytostatic effect |
| | T/C_{corr} | or | τ < 0 % | cytocide effect |

### Results

### a) Concentration dependent test (IC₅₀)

**Table 3: IC₅₀-values of Fe(II)salophen, Fe(III)salophen chloride, o-phenylenediamine and Fe(OAc)₂ resp. FeCl₃ on MCF-7 and MDA-MB-231 cells**

| Compound | MCF-7 (µM) | MDA-MB 231 (µM) |
|---|---|---|
| Fe(OAc)₂ | > 50 | > 50 |
| FeCl₃ | >100 | > 100 |
| o-phenylenediamine | 5.1 (±0.70) | 23.5 (±2.40) |
| Fe(II)salophen | 0.4 (±0.07) | 0.1 (±0.01) |
| Fe(III)salophen chloride | 0.3 (±0.01) | 0.01 (±0.01) |

**Table 4: IC₅₀ values of established cytostic agents in MCF-7 and MDA-MB-231 cells**

| Compound | MCF-7(µM) | MDA-MB-231(µM) |
|---|---|---|
| cisplatin | 2.0 (±0.3) | 4.0 (±1.5) |
| tamoxifen | 2.3 (±0.4) | 10.6 (±0.3) |
| 5-fluorouracil | 4.8 (±0.6) | 9.6 (±0.3) |
| Melphalan | 5.7 (±1.5) | 3.9 (±0.3) |
| Co-Ass¹ | 1.4 (± 0.3) | 1.9 (± 0.3) |

| | | |
|---|---|---|
| ¹ Hexacarbonyl[2-propyn-1-ylacetylsalicylat]dicobalt | | |

At it is apparent from tables 3 and 4, the iron complexes of the invention show cytotoxic effects at considerably lower concentrations in comparison to their ligand and to established cytostatic agents.

### b) Time dependent test

### 1.) Time dependent test on MCF-7 cells

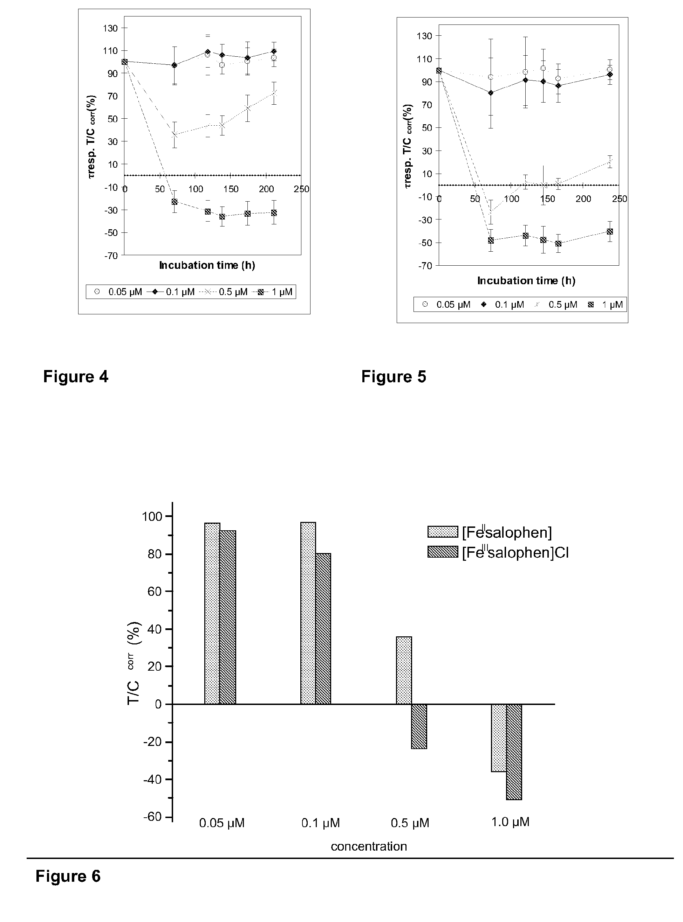

Figures 4 and 5 show the results for Fe(II)salophen and Fe(III)salophen chloride, respectively. Table 5 and figure 6 summarize the results on MCF-7 cells. Figure 6 exhibits the antiproliferative activity of the [Fe^{II/III}salophen] complexes of the invention on MCF-7-cells using a bar chart.

**Table 5: Minimum T/C_{corr}- resp. τ-values (time) on MCF-7-cells: [Fe^{II/III}salophen] complexes and Cisplatin as reference**

| | **minimum T/C_{corr}- resp. τ-value (%)** | | | |
|---|---|---|---|---|
| | **0.05 µM (h)** | **0.1 µM (h)** | **0.5 µM (h)** | **1 µM (h)** |
| Cisplatin | - | - | 55.1 (172) | 2.2 (172) |
| [Fe^{II}salophen] | 96.5(71) | 96.7(71) | 35.7 (71) | -35.9 (138) |
| [Fe^{III}salophen]Cl | 92.6 (166) | 80.4 (72) | -23.4 (72) | -50.8 (166) |

The time dependent test results show that the Fe(III)salophen chloride complex is still more effective on MCF-7 cells than the Fe(II)salophen complex. At a concentration of 0.5 µM Fe(III)salophen chloride already shows strong cytocide effects. As apparent from table 5, the complexes of the invention are considerably more effective than the established cytostatic agent cisplatin.

### 2.) Time dependent test on MDA-MB-231 cells

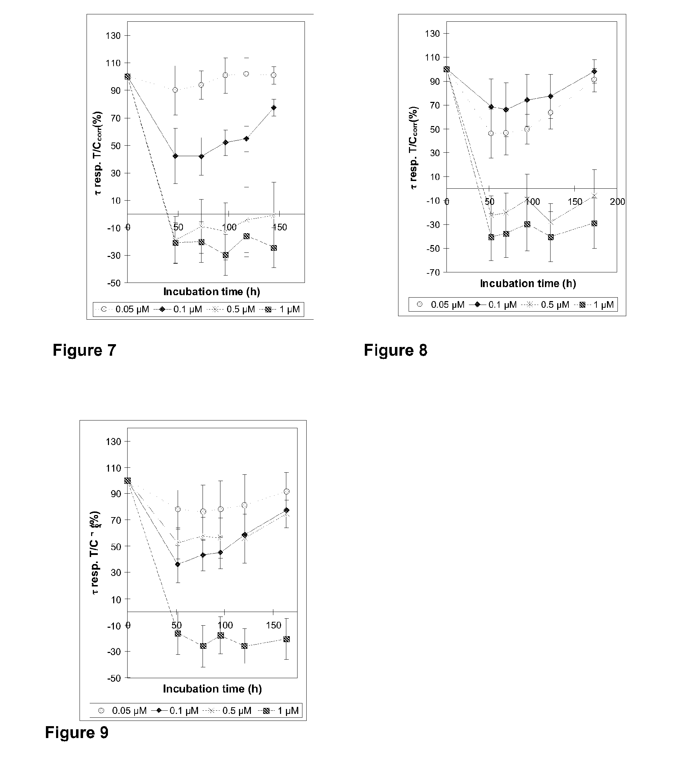

Figure 7 exhibits the results for Fe(II)salophen. Figure 8 shows the results for Fe(III)salophen chloride and table 6 gives a summary.

**Table 6: Minimum T/C_{corr}- resp. τ-values (time) on MDA-MB-231-cells: [Fe^{II/III}salophen] complexes and Cisplatin as reference**

| | **minimum T/C_{corr}- resp. τ-value (%)** | | | |
|---|---|---|---|---|
| | **0.05 µM (h)** | **0.1 µM (h)** | **0.5 µM (h)** | **1 µM (h)** |
| Cisplatin | - | - | 79.7 (72) | 69.0 (72) |
| [Fe^{II}salophen] | 90.0 (49) | 41.9 (71) | -19.1 (47) | -29.7 (47) |
| [Fe^{III}salophen]Cl | 46.0 (53) | 66.2 (69) | -27.8 (122) | -40.8 (53) |

Again, both iron complexes show a considerably stronger effectiveness on MDA-MB-231 cells than the established cytostatic agent cisplatin.

### 3.) Time dependent test on HeLa cells

Figure 9 exhibits the results for Fe(III)salophen chloride and table 7 gives a summary.

**Table 7: Minimum T/C_{corr}- resp. τ-values (time) on HeLa cells: Fe^{III}[salophen]Cl and Cisplatin as reference.**

| | **minimum T/C_{corr}- resp. τ-value (%)** | | |
|---|---|---|---|
| | **0.1 µM(h)** | **0.5 µM (h)** | **1** µ**M (h)** |
| Cisplatin | - | 55.9 (72) | 33.5 (91) |
| [Fe^{III}salophen]Cl | 36.2 (51) | 52.1 (51) | -26.0 (77) |

The superiority of Fe(III)salophen chloride beyond cisplatin is evident.

### 4.) Time dependent test on LNCaP-FGC-cells

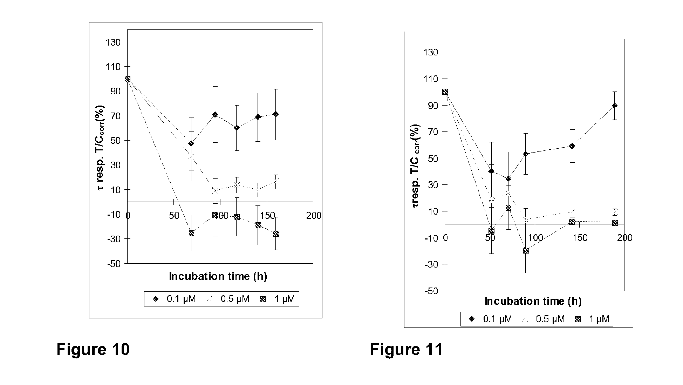

Figure 10 exhibits the results for Fe(II)salophen and figure 11 for Fe(III) salophen chloride. Table 8 summarizes results.

**Table 8: Minimum T/C_{corr}- resp. τ-values (time) on LNCaP cells: [Fe^{II/III}salophen] complexes and Cisplatin, Carbolatin und Oxaliplatin as references.**

| | **minimum T/C_{corr}- resp. τ-value (%)** | | | |
|---|---|---|---|---|
| | **0.1 µM (h)** | **0.5 µM (h)** | **1 µM (h)** | **5 µM (h)** |
| Cisplatin | - | 59.0 (98) | 32.7 (124) | 6.7(124) |
| Carboplatin | - | 69.5 (73) | 55.5 (46) | 19.6 (73) |
| Oxaliplatin | - | 12.7 (124) | -0.6 (124) | -31.0 (98) |
| [Fe^{II}salophen] | 47.3 (69) | 8.9 (95) | -25.6 (160) | - |
| [Fe^{III}salophen]Cl | 34.4 (71) | 3.6 (90) | -19.5 (90) | - |

The compounds of the invention show a considerably stronger cytotoxic effect that the platin-containing cytostatic agents cisplatin and carboplatin.

## Claims

1. Metal salophen complexes of general formulas I and/or II 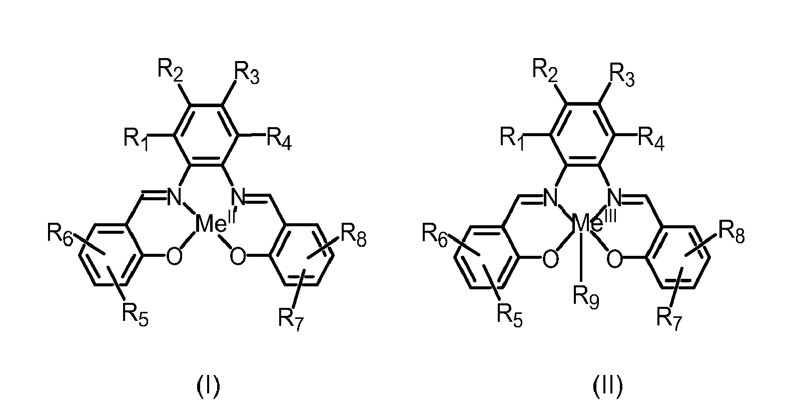 wherein
Me(II) represents Fe, Co, Ni, Cu, Mn, Zn
Me(III) represents Fe, Mn, Al, Cr
R₁ to R₈ independently from each other represent hydrogen, halogen, hydroxy, C₁-C₄-alkyl, C₁-C₄-alkoxy, trihalomethyl
R₉ represents halogen or acetat
or their salts or esters
for use in prevention and treatment of cancer.

2. Metal salophen complex of claim 1, wherein Me(II) represents Fe(II) or Me(III) represents Fe(III).

3. Metal salophen complex of claim 1 or 2, wherein R₉ represents chlorine, fluorine or bromine, preferably chlorine.

4. Metal salophen complex of claim 1, wherein halogen in R₁ to R₈ represents fluorine, chlorine, bromine or iodine, preferably fluorine.

5. Metal salophen complex of claim 1, wherein C₁-C₄-alkoxy is methoxy or ethoxy.

6. Metal salophen complex of claim 1, wherein trihalomethyl is trifluoromethyl or trichloromethyl.

7. Use of a metal salophen complex of general formulas I and/or II, 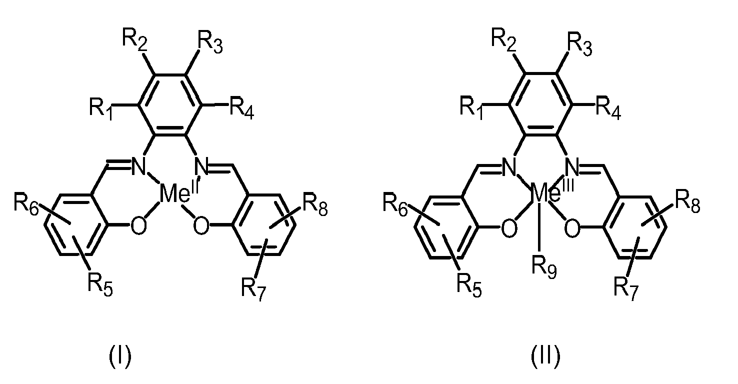 wherein
Me(II) represents Fe, Co, Ni, Cu, Mn, Zn
Me(III) represents Fe, Mn, Al, Cr
R₁ to R₈ independently from each other represent hydrogen, halogen, hydroxy, C₁-C₄-alkyl, C₁-C₄-alkoxy, trihalomethyl
R₉ represents halogen or acetat
or their salts or esters
for the manufacture of a pharmaceutical composition for prevention or treatment of cancer.

8. Use according to claim 7, wherein Fe(II)salophen is used.

9. Use according to claim 7, wherein Fe(III)salophen chloride is used.

10. Use according to claim 7 for the prevention or treatment of breast, colorectal, lungs, gastric, pancreatic or esophagal cancer and their metastases, preferably breast cancer.

11. Use according to claim 7 for the prevention or treatment of a neoplasia, preferably leukaemia.

12. A pharmaceutical composition comprising as active substance a metal salophen complex of general formulas I and/or II, 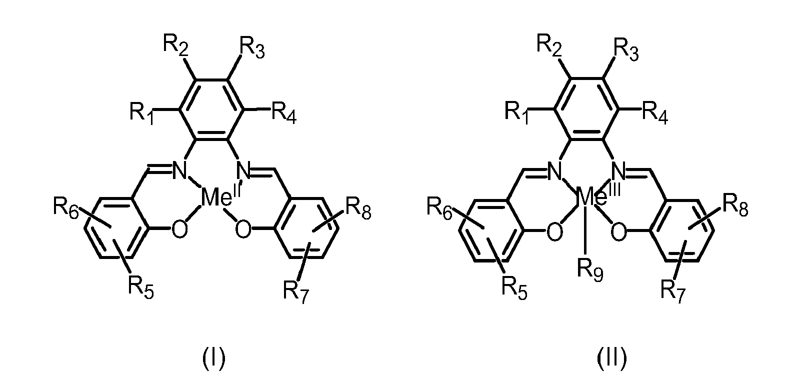 wherein
Me(II) represents Fe, Co, Ni, Cu, Mn, Zn
Me(III) represents Fe, Mn, Al, Cr
R₁ to R₈ independently from each other represent hydrogen, halogen, hydroxy, C₁-C₄-alkyl, C₁-C₄-alkoxy, trihalomethyl
R₉ represents halogen or acetat
or their salts or esters.

13. A method for treating cancer which comprises adminstering to a patient suffering from cancer a therapeutically effective amount of a metal salophen complex of general formulas I and/or II, 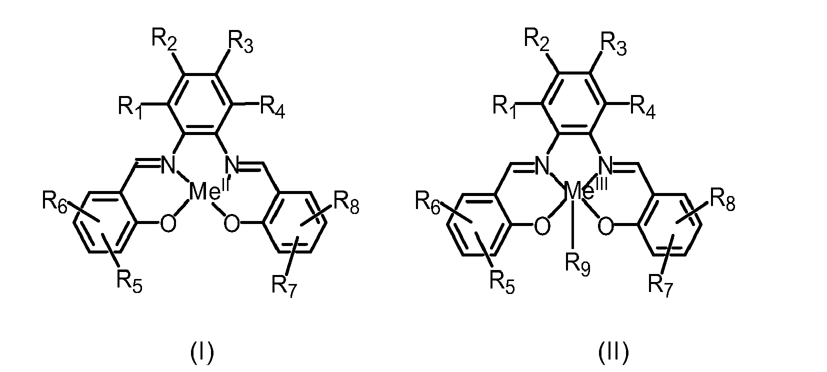 wherein
Me(II) represents Fe, Co, Ni, Cu, Mn, Zn
Me(III) represents Fe, Mn, Al, Cr
R₁ to R₈ independently from each other represent hydrogen, halogen, hydroxy, C₁-C₄-alkyl, C₁-C₄-alkoxy, trihalomethyl
R₉ represents halogen or acetat
or their salts or esters.
